(19) Europäisches Patentamt
European Patent Office
Office européen des brevets

(11) Numéro de publication: 0 148 042
B1

(12) # FASCICULE DE BREVET EUROPÉEN

(45) Date de publication du fascicule du brevet: **20.05.87**

(51) Int. Cl.⁴: **C 07 D 495/04,** A 61 K 31/40 // (C07D495/04, 333:00, 209:00)

(21) Numéro de dépôt: **84402253.3**

(22) Date de dépôt: **08.11.84**

(54) **Dérivés de thiéno (2,3-b)pyrrole,leur procédé de préparation et les compositions pharmaceutiques les renfermant.**

(30) Priorité: **10.11.83 FR 8317866**

(43) Date de publication de la demande: **10.07.85 Bulletin 85/28**

(45) Mention de la délivrance du brevet: **20.05.87 Bulletin 87/21**

(84) Etats contractants désignés: **AT BE CH DE FR GB IT LI LU NL SE**

(56) Documents cité:
**FR-A-2 537 974**
**FR-M-8 439**

**A. BURGER Medicinal Chemistry Third Edition, Part-I p. 77**

**Le dossier contient des informations techniques présentées postérieurement au dépôt de la demande et ne figurant pas dans le présent fascicule.**

(73) Titulaire: **ADIR, 22, rue Garnier, F-92200 Neuilly- sur- Seine (FR)**

(72) Inventeur: **Wierzbicki, Michel, 32 rue Lucien Voilin, F-92800 Puteaux (FR)**

(74) Mandataire: **Reverbori, Marcelle, ADIR 22 Rue Garnier, F-92200 Neuilly- sur- Seine (FR)**

EP 0 148 042 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

La présente invention a pour objet de nouveaux dérivés de thiéno [2,3-b] pyrrole, leur procédé de préparation et les compositions pharmaceutiques les renfermant.

Elle concerne les dérivés de thiéno [2,3-b] pyrrole de formule générale I:

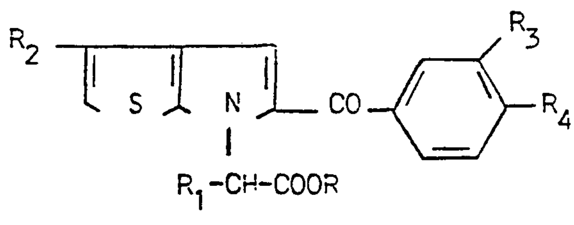

(I)

dans laquelle:

R représente un atome d'hydrogène, un radial alcoyle, en chaîne droite ou ramifiée, ayant de 1 à 5 atomes de carbone, un métal alcalin ou alcalino, terreux, ou un radical ammonium, mono-, di-ou trialcoylammonium dans lequel le groupe alcoyle renferme de 1 à 5 atomes de carbone;

$R_1$ représente un atome d'hydrogène ou un radical alcoyle contenant de 1 à 5 atomes de carbone en chaîne droite ou ramifiée;

$R_2$ représente un atome d'hydrogène, un radical alcoyle en chaîne droite ou ramifiée ayant de 1 à 5 atomes de carbone, un radical phenyle ou un radical phénylalcoyle dans lequel le groupe alcoyle contient de 1 à 5 atomes de carbone en chaîne droite ou ramifiée;

$R_3$ et $R_4$, identiques ou différents, représentent chacun un atome d'hydrogène ou d'halogène ou un radical alcoyle ou alcoxy ayant chacun de 1 à 5 atomes de carbone en chaîne droite ou ramifiée, ou

$R_3$ et $R_4$ représentent ensemble un radical de formule -CH=CH-CH=CH- de façon à former avec le groupe phényle auquel ils sont liés un groupe naphtyle.

L'état antérieur de la technique est illustré notamment par le brevet spécial de Médicament français 8439M lequel décrit des 2-méthyl indoles substitués de formule:

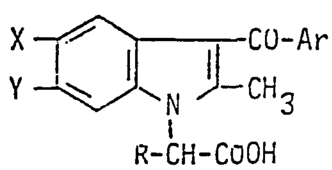

ayant la double activité anti-inflammatoire et analgésique. Les différences de structure entre les dérivés de la présente demande et les dérivés indoliques antérieurement connus conduisent aux thiéno [2,3-b] pyrroles objet de la présente demande qui sont eux essentiellement analgésique, ne présentent donc pas les effets secondaires néfastes des anti-inflammatoires, et de plus ne sont pas métabolisés comme l'est par exemple la clométacine produit commercial considéré comme le plus proche des présents dérivés.

La présente invention a également pour objet le procédé de préparation des dérivés de formule générale I caractérisé en ce que:

l'on traite le dérivé de formule générale II:

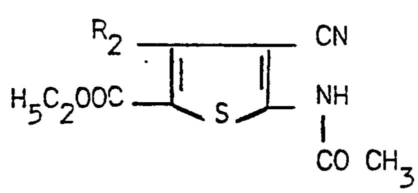

(II)

dans laquelle $R_2$ a la signification énoncée ci-dessus, avec un dérivé de formule générale III

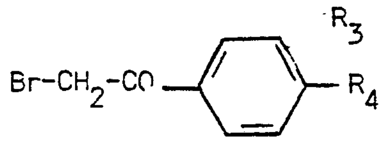

(III)

dans laquelle $R_3$ et $R_4$ ont les significations précédemment énoncées, en présence de $K_2CO_3$ et d'acétone pour obtenir le dérivé de formule générale IV:

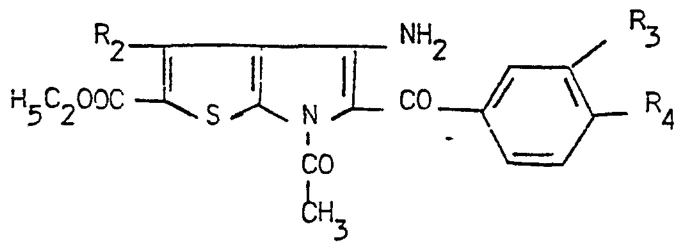

(IV)

dans laquelle $R_2$, $R_3$ et $R_4$ sont tels que précédemment définis; lequel composé IV est traité par $KNO_2$, $HCl/H_2O$ et $CH_3OH$ pour obtenir le composé de formule générale V:

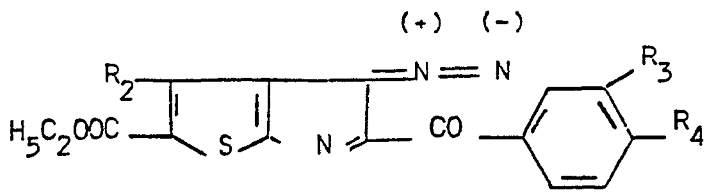

(V)

dans laquelle $R_2$, $R_3$ et $R_4$ ont les significations précédemment définies,

lequel est dédiazoté au moyen d'azabisisobutyronitrile en milieu éthanol en présence d'acide en un composé de formule générale VI:

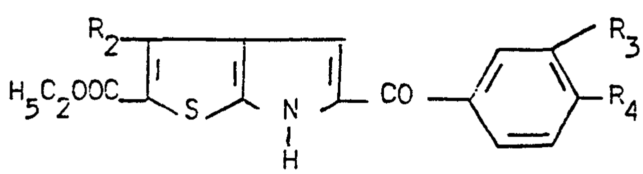

(VI)

dans laquelle $R_2$, $R_3$ et $R_4$ sont tels que précédemment définis, que l'on traite tout d'abord par $NaOH/H_2O/C_2H_5OH$ puis par $HCl/H_2O$ pour donner le composé de formule générale VII:

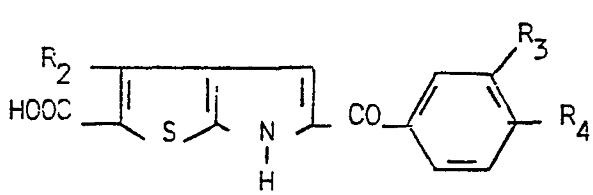

(VII)

dans laquelle $R_2$, $R_3$ et $R_4$ sont tels que précédemment définis, que l'on chauffe en présence de cuivre et de quinoléine pour obtenir le composé de formule générale VIII:

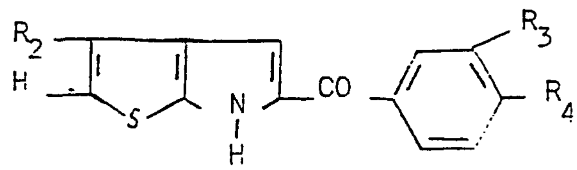

(VIII)

dans laquelle $R_2$, $R_3$ et $R_4$ ont les significations énoncées précédemment, que l'on traite, en présence de $C_2H_5ONa/C_2H_5OH$, par un dérivé de formule générale IX:

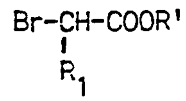

(IX)

dans laquelle $R_1$ prend la signification énoncée précédemment et R' a la signification de R à l'exception d'un atome d'hydrogène,
pour obtenir le dérivé de formule générale I':

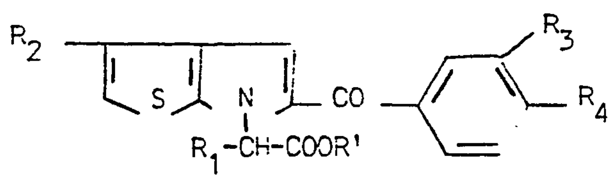

(I')

dans laquelle R', $R_1$, $R_2$, $R_3$ et $R_4$ sont tels que précédemment définis, lequel dérivé (I') est traité par $NaOH/H_2O/C_2H_5OH$, puis une solution d'acide chlorhydrique pour obtenir le dérivé de formule générale I":

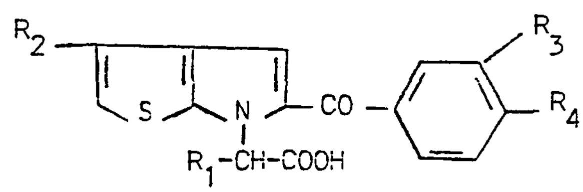

(I")

dans laquelle $R_1$, $R_2$, $R_3$ et $R_4$ ont les significations énoncées précédemment.

L'ensemble des dérivés (I') et (I") forme l'ensemble des derivés de formule générale I.

Les produits de départ de formule générale II ont été préparés selon la méthode de Gewald K et coll, Chem. Ber. (1966), 99 p.94 et 2712 à partir de matières premières connues.

Les dérivés de formule générale I possèdent des propriétes pharmacologiques intéressantes, notamment des propriétés analgésiques et à un degré moindre des propriétés antiinflammatoires.

Leur toxicité est faible et leur $DL_{50}$ déterminée per os chez la souris est supérieure à 1000mg/kg.

L'activité analgésique des dérivés de l'invention a été mise en évidence notamment par le test de Hendershot, L.C, Forsaith, J.J., J. Pharmacol. Exp. Ther. (1959), 125, 237, relatif aux crampes abdominales induites à la phénylbenzoquinone, et par le test de Koster R, Anderson M., de Beer E.S., Fed. Proc. (1959), 18, 412, relatif aux étirements à l'acide acétique. Pour chacun de ces tests, la dose efficace moyenne $DE_{50}$ se situe, pour les dérivés de l'invention, entre 50 et 200mg/kg en administration orale chez la souris.

Les propriétés pharmacologiques ci-dessus décrites ainsi que la faible toxicité des dérivés de formule générale I permettent leur utilisation en thérapeutique principalement dans le traitement des douleurs aigües ou chroniques et notamment des douleurs accompagnant un processus inflammatoire (douleurs rhumatismales d'arthrose, d'arthrite, de lombo-sciatique etc...), des douleurs traumatiques, post-traumatiques ou postopératoires, des douleurs des sphères ORL, stomatologique ou génito-urinaire, et également de certaines céphalées, nevralgies, migraines et douleurs cancéreuses.

La présente invention a également pour objet les compositions pharmaceutiques contenant comme principe actif un composé de formule générale I mélangé ou associé à un excipient pharmaceutique approprié, comme

par exemple, l'eau distillée, le glucose, le lactose, l'amidon, le talc, l'éthyl cellulose le stéarate de magnésium ou le beurre de cacao.

Les compositions pharmaceutiques ainsi obtenues se présentent généralement sous forme dosée et peuvent contenir de 25 à 250 mg de principe actif. Elles peuvent revêtir par exemple la forme de comprimés, dragées, gélules, suppositoires, solutions injectables ou buvables, pommades et être, selon les cas, administrées par voie orale, rectale, parentérale ou locale à la dose de 25 à 250mg 1 à 4 fois par jour.

Les exemples suivants illustrent l'invention, les points de fusion étant, sauf mention contraire, déterminés à la platine chauffante de Kofler.

**Exemple 1**

méthyl-3 benzoyl-5 carboxyméthyl-6 thiéno [2,3-b] pyrrole

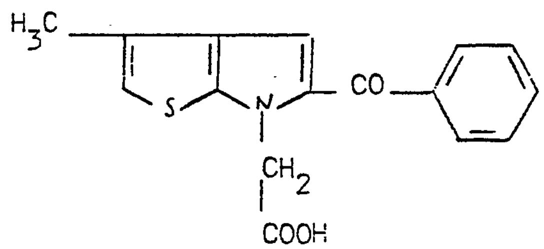

250g. d'acétamido-2 cyano-3 méthyl-4 éthoxycarbonyl-5 thiophène préparés selon K. Gewald sont portés à reflux dans 1500ml d'acétone avec 250g. d'α-bromo acétophénone et 220g de carbonate de potassium pendant 24 heures; le tout est alors précipité dans 4000ml d'eau glacée. Le précipité formé est filtré, lavé avec 1000ml d'un mélange glacé eau-éthanol (1/1) puis séché et enfin lavé avec 1000ml d'un mélange froid cyclohexane benzène (9/1). On obtient ainsi 295g d'éthoxycarbonyl-2 méthyl-3 amino-4 benzoyl-5 acétyl-6 thiéno [2,3-b] pyrrole, P.F.: 188° C.

De la même façon ont été obtenus:
- l'éthoxycarbonyl-2 méthyl-3 amino-4 p.méthylbenzoyl-5 acétyl-6 thiéno [2,3-b] pyrrole, P.F.: 198-199° C;
- l'éthoxycarbonyl-2 propyl-3 amino-4 benzoyl-5 acétyl-6 thiéno [2,3-b] pyrrole, P.F.: 149° C;
- l'éthoxycarbonyl-2 méthyl-3 amino-4 (diméthoxy-3,4 benzoyl)-5 acétyl-6 thiéno [2,3-b] pyrrole, P.F.: 204° C;
- l'éthoxycarbonyl-2 méthyl-3 amino-4 (naphtoyl-2)-5 acétyl-6 thiéno [2,3-b] pyrrole, P.F.: 220-221° C;
- l'éthoxycarbonyl-2 méthyl-3 amino-4 p.chlorobenzoyl-5 acétyl-6 thiéno [2,3-b] pyrrole, P.F.: 140° C;
- l'éthoxycarbonyl-2 méthyl-3 amino-4 p.méthoxybenzoyl-5 acétyl-6 thiéno [2,3-b] pyrrole, P.F.: 201° C.

L'éthoxycarbonyl-2 méthyl-3 amino-4 benzoyl-5 acétyl-6 thiéno [2,3-b] pyrrole obtenu, est mis en suspension dans 2000ml de méthanol entre 25 et 30° C, et on additionne successivement des fractions de 25ml de HCl (4N) et de 8,5g de nitrite de potassium dans 20ml d'$H_2O$ jusqu'à disparition du produit de départ ou de son analogue déacétylé. Le mélange réactionnel est alors filtré, lavé avec un mélange eau-methanol 1/1 et de produit obtenu utilisé tel quel pour la suite est: l'éthoxycarbonyl-2 méthyl-3 diazo-4 benzoyl-5 [4H]-thiéno [2,3-b] pyrrole. Un echantillon recristallisé fond à 180° C.

Le produit obtenu est additionné à 1500ml d'éthanol et 5ml d'$H_2SO_4$ à 10 %, on porte à reflux en additionnant de temps à autre une quantité catalytique d'azabisisobutyronitrile (ABIN), et en distillant l'acétaldéhyde formé. Une fois le dégagement d'azote terminé, on refroidit, évapore l'éthanol sur un évaporateur rotatif et sublime l'isobutyronitrile formé sous pression réduite. On récupère ainsi 200g d'éthoxycarbonyl-2 méthyl-3 benzoyl-5 thiéno [2,3-b] pyrrole utilisable tel quel, un échanteillon recristallisé fond à 180-181° C.

De la même façon ont été obtenus:
- l'ethoxycarbonyl-2 méthyl-3 p.méthoxybenzoyl-5 thiéno [2,3-b] pyrrole, P.F.: 198° C;
- l'éthoxycarbonyl-2 propyl-3 benzoyl-5 thiéno [2,3-b] pyrrole, P.F.: 151° C;
- l'éthoxycarbonyl-2 méthyl-3 (diméthoxy-3,4 benzoyl)-5 thiéno [2,3-b] pyrrole, P.F.: 270-272° C;
- l'éthoxycarbonyl-2 méthyl-3 (naphtoyl-2)-5 thiéno [2,3-b] pyrrole, P.F.: 189-190° C;
- l'éthoxycarbonyl-2 méthyl-3 p.chlorobenzoyl-5 thiéno [2,3-b] pyrrole, P.F.: 195° C
- l'éthoxycarbonyl-2 méthyl-3 p.méthoxybenzoyl-5 thiéno [2,3-b] pyrrole, P.F.: 155° C.

Les 200g d'éthoxycarbonyl-2 méthyl-3 benzoyl-5 thiéno [2,3-b] pyrrole précédemment obtenus sont portés à reflux dans un mélange de 60g de NaOH 1500ml d'eau et 1500ml d'éthanol jusqu'à hydrolyse totale (environ 15 heures). On distille ensuite l'éthanol, refroidit le milieu, filtre; le filtrat est acidifié à pH voisin de 3 et filtré. Le produit obtenu est lavé à l'eau puis séché et utilisé tel quel (rendement à peu près quantitatif).

L'acide ainsi obtenu est mélangé à 1200ml de quinoléine. On additionne 2g de poudre de cuivre et on chauffe; la décarboxylation se produit vers 200-210° C, on laisse la température s'élever jusqu'à l'ébullition. On

refroidit, hydrolyse dans une quantité stoechiométrique d'acide chlorhydrique (4 N) additionné de glace. Le précipité obtenu est filtré, lavé avec HCl (N) puis recristallisé dans l'éthanol.

On obtient ainsi 145g de:

méthyl-3 benzoyl-5 thiéno [2,3-b] pyrrole P.F. = 199° C.

De la même façon ont été obtenus:

- le méthyl-3 p.méthylbenzoyl-5 thiéno [2,3-b] pyrrole, P.F.: 256° C;
- le propyl-3 benzoyl-5 thiéno [2,3-b] pyrrole, P.F.: 155° C;
- le méthyl-3 (diméthoxy-3,4.benzoyl)-5 thiéno [2,3-b] pyrrole, P.F.: 191° C;
- le méthyl-3 (naphtoyl-2)-5 thiéno [2,3-b] pyrrole, P.F.: 179° C;
- le méthyl-3 p.chlorobenzoyl-5 thiéno [2,3-b] pyrrole, P.F.: 233° C;
- le méthyl-3 p.méthoxybenzoyl-5 thiéno [2,3-b] pyrrole, P.F.: 198° C.

Le méthyl-3 benzoyl-5 thiéno [2,3-b] pyrrole précédemment obtenu, mis en suspension dans 500 ml d'éthanol est additionné à une solution d'éthylate de sodium fraîchement préparée par dissolution de 24,6g de sodium dans 500ml d'éthanol, on laisse sous agitation pendant 15 minutes puis additionne 125g de bromacétate d'éthyle. On porte 4 heures à reflux puis hydrolyse par addition de 600ml de solution de soude (N). On porte à reflux 30 minutes et élimine l'éthanol par distillation. La solution refroidie est alors filtrée. Le filtrat acidifié à pH voisin de 3, le précipité obtenu, lavé à l'eau, est recristallisé dans un mélange benzènecyclohexane. On obtient alors 150g de:

méthyl-3 benzoyl-5 carboxyméthyl-6 thiéno [2,3-b] pyrrole P.F. = 197° C.

**Exemples 2-8**

Les dérivés suivants ont, été obtenus selon le procédé décrit dans l'exemple 1, en remflacant pour les produits des exemples 2, 4, 5, 6, 7, et 8 le broms-acétate d'éthyle par le 2-bromo-propionate d'éthyle.

2) méthyl-3 benzoyl-5 α-carboxyéthyl-6 thiéno, [2,3-b] pyrrole, ν CO: 1620 $cm^{-1}$ et 1720 $cm^{-1}$.

3) méthyl-3 p.méthoxybenzoyl-5 carboxyméthyl-6 thiéno [2,3-b] pyrrole, P.F. = 144° C.

4) méthyl-3 p.chlorobenzoyl-5 α-carboxyéthyl-6 thiéno [2,3-b] pyrrole, P.F. = 117-118° C, P.F. du sel de sodium correspondant > 250° C.

5) méthyl-3 (diméthoxy-3,4 benzoyl)-5 α-carboxyéthyl-6 thiéno [2,3-b] pyrrole, F = 199° C.

6) n.propyl-3 benzoyl-5 α-carboxyéthyl-6 thiéno [2,3-b] pyrrole, ν CO: 1610 $cm^{-1}$ et 1730 $cm^{-1}$

7) méthyl-3 p.méthylbenzoyl-5 α-carboxyéthyl-6 thiéno [2,3-b] pyrrole, ν CO: 1610 $cm^{-1}$ et 1730 $cm^{-1}$.

8) méthyl-3 (napthoyl-2)-5 α-carboxyéthyl-6 thiéno [2,3-b] pyrrole, ν CO: 1615 et 1725 $cm^{-1}$.

**Revendications**

pour les Etats contractants BE, CH, FR, GB, IT, LT, LU, NL, SE

1) Les dérivés de thiéno [2,3-b] pyrrole de formule générale I:

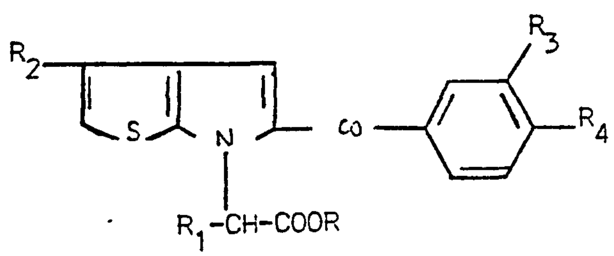

(I)

dans laquelle:

R représente un atome d'hydrogène, un radical alcoyle en chaîne droite ou ramifiée ayant de 1 à 5 atomes de carbone, un métal alcalin ou alcalinoterreux, ou un radical ammonium, mono- di- ou tri- alcoylammonium dans lequel le groupe alcoyle renferme de 1 à 5 atomes de carbone;

$R_1$ représente un atome d'hydrogène, ou un radical alcoyle contenant de 1 à 5 atomes de carbone, en chaîne droite ou ramifiée;

$R_2$ représente un atome d'hydrogène, un radical alcoyle en chaîne droite ou ramifiée ayant de 1 à 5 atomes de carbone, un radical phényle ou un radical phénylalcoyle dans lequel le groupe alcoyle contient de 1 à 5 atomes de carbone en chaîne droite ou ramifiée;

$R_3$ et $R_4$, identiques ou différents, représentent chacun un atome d'hydrogène ou d'halogène ou un radical

alcoyle ou alcoxy ayant chacun de 1 à 5 atomes de carbone en chaîne droite ou ramifiée, ou

$R_3$ et $R_4$ représentent ensemble un radical de formule: -CH=CH-CH=CH- de façon à former avec le groupe phényle auquel ils sont liés un groupe naphtyle.

2) le méthyl-3 benzoyl-5 carboxyméthyl-6 thiéno [2,3-b] pyrrole.

3) le méthyl-3 benzoyl-5 α-carboxyéthyl-6 thiéno [2,3-b] pyrrole.

4) le méthyl-3 p.méthoxybenzoyl-5 carboxyméthyl-6 thiéno [2,3-b] pyrrole.

5) méthyl-3 p.chlorobenzoyl-5 α-carboxyéthyl-6 thiéno [2,3-b[ pyrrole.

6) le procédé de préparation des composés de la revendication 1 caractérisé en ce que:

l'on traite le dérivé de formule générale II:

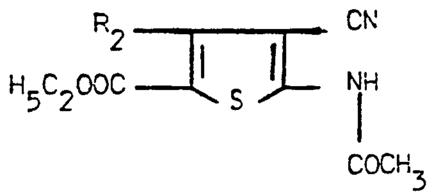

(II)

dans laquelle $R_2$ à la signification énoncée dans la revendication 1,

avec un dérivé de formule générale III:

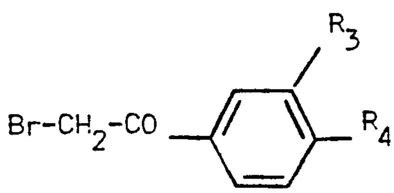

(III)

dans laquelle $R_3$ et $R_4$ ont les significations énoncées dans la revendication 1, en présence de $K_2CO_3$ et d'acétone pour obtenir le dérivé de formule générale IV.

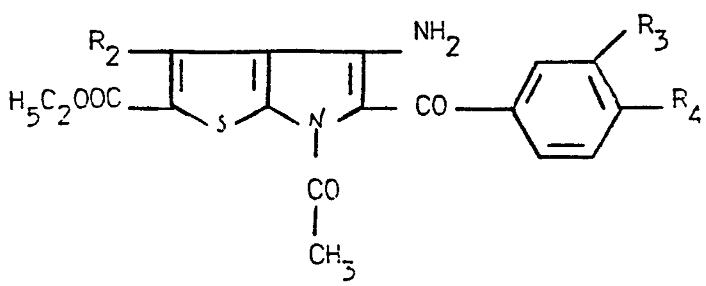

(IV)

dans laquelle $R_2$, $R_3$ et $R_4$ sont tels que définis dans la revendication 1,

lequel compose IV est traité par $KNO_2$, $HCl/H_2O$ et $CH_3OH$ pour obtenir le composé de formule générale V:

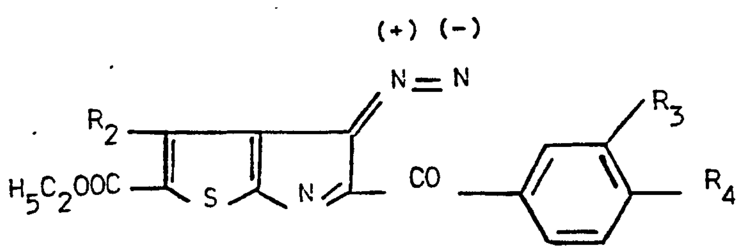

(V)

dans laquelle $R_2$, $R_3$ et $R_4$ ont les significations définies dans la revendication 1,

lequel est dédiazoté au moyen d'azabisisobutyronitrile en milieu éthanol en présence d'acide en un composé

de formule générale VI:

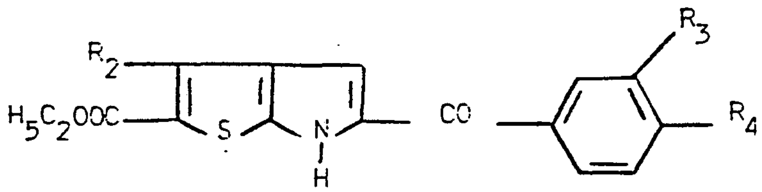

(VI)

dans laquelle $R_2$, $R_3$ et $R_4$ sont tels que définis dans la revendication 1,

que l'on traite tout d'abord par $NaOH/H_2O/C_2H_5OH$ puis par $HCl/H_2O$ pour donner le composé de formule générale VII:

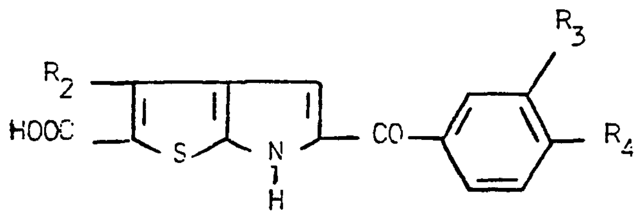

(VII)

dans laquelle $R_2$, $R_3$ et $R_4$ sont tels que définis dans la revendication 1, que l'on chauffe en présence de cuivre et de quinoléine pour obtenir le dérivé de formule générale VIII:

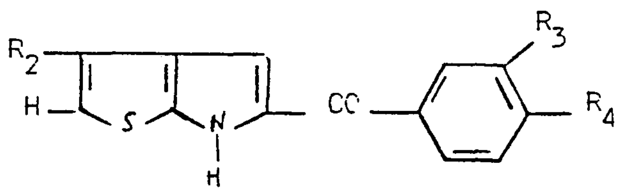

(VIII)

dans laquelle $R_2$, $R_3$ et $R_4$ ont les significations énoncées dans la revendication 1,

que l'on traite en présence de $C_2H_5ONa/C_2H_5OH$, par un dérivé de formule générale IX:

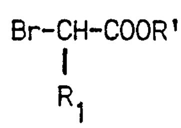

(IX)

dans laquelle $R_1$ a la signification énoncée dans la revendication 1 et R' a la signification de R énoncée dans la revendication 1 à l'exception d'un atome d'hydrogène,

pour obtenir le dérivé de formule générale I':

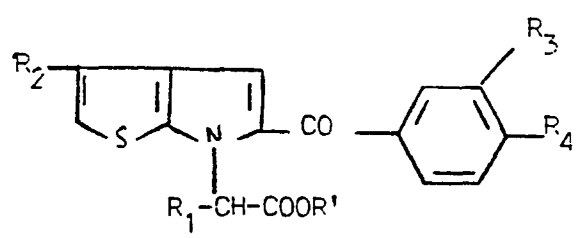

(I')

dans laquelle R', $R_1$, $R_2$, $R_3$ et $R_4$ sont tels que prédémment définis,

lequel dérivé (I') est traité par $NaOH/H_2O/C_2H_5OH$ puis par une solution d'acide chlorhydrique pour obtenir le dérivé de formule générale I'':

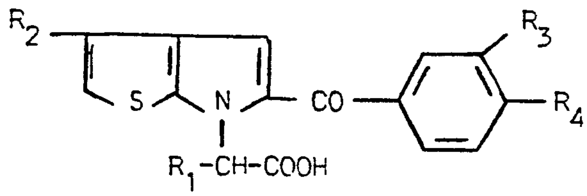

(I")

dans laquelle $R_1$, $R_2$, $R_3$ et $R_4$ sont tels que définis dans la revendication 1.

7) Les compositions pharmaceutiques contenant comme principe actif un composé selon les revendications 1 à 5, avec des excipients appropriés.

8) Les compositions pharmaceutiques selon la revendication 7 pour utilisation dans le traitement des douleurs aigües ou chroniques.

**Revendication**

pour l'etat contractant AT

Procédé de préparation des dérivés de thiéno [2,3-b] pyrrole de formule générale I:

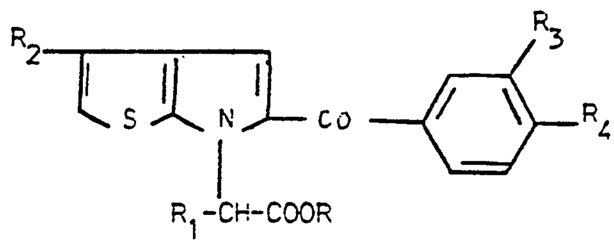

(I)

dans laquelle:

R représente un atome d'hydrogène, un radical alcoyle en chaîne droite ou ramifiée ayant de 1 à 5 atomes de carbone, un métal alcalin ou alcalinoterreux, ou un radical ammonium, mono- di- ou trialcoylammonium dans lequel le groupe alcoyle renferme de 1 à 5 atomes de carbone;

$R_1$ représente un atome d'hydrogène, ou un radical alcoyle contenant de 1 à 5 atomes de carbone, en chaîne droite ou ramifiée;

$R_2$ représente un atome, d'hydrogène, un radical alcoyle en chaîne droite ou ramifiée ayant de 1 à 5 atomes de carbone, un radical phényle ou un radical phénylalcoyle dans lequel le groupe alcoyle contient de 1 à 5 atomes de carbone en chaîne droite ou ramifiée;

$R_3$ et $R_4$, identiques ou différents, représentent chacun un atome d'hydrogène ou d'halogène ou un radical alcoyle ou alcoxy ayant chacun de 1 à 5 atomes de carbone en chaîne droite ou ramifiée, ou $R_3$ et $R_4$ représentent ensemble un radical de formule: -CH=CH-CH=CH- de façon à former avec le groupe phényle auquel ils sont liés un groupe naphtyle;

caractérisé en ce que:

l'on traite le dérivé de formule générale II:

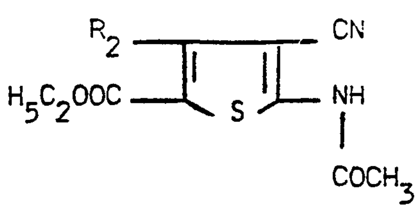

(II)

dans laquelle $R_2$ a la signification énoncée ci-dessus avec un dérivé de formule générale III:

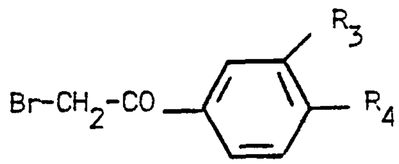

(III)

dans laquelle $R_3$ et $R_4$ ont les significations énoncées précédemment en présence de $K_2CO_3$ et obtenir le dérivé de formule générale IV:

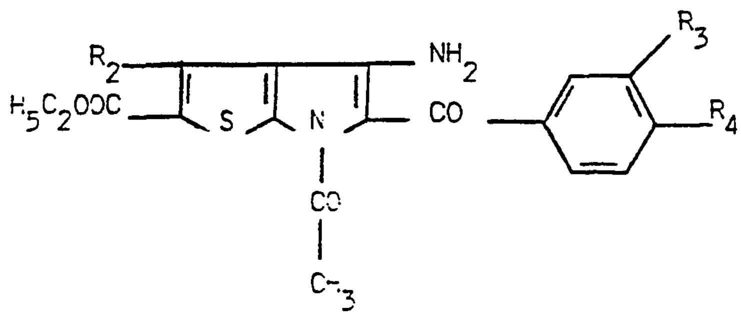

(IV)

dans laquelle $R_2$, $R_3$ et $R_4$ sont tels que précédemment définis,
lequel composé IV est traité par $KNO_2$, $HCl/H_2O$ et $CH_3OH$ pour obtenir le composé de formule générale V:

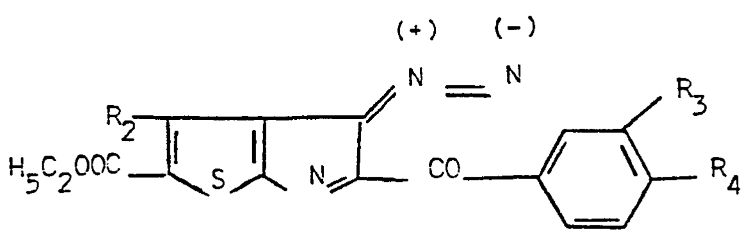

(V)

dans laquelle $R_2$, $R_3$ et $R_4$ ont les significations définies précédemment,
lequel est dédiazoté au moyen d'azabisisobutyronitrile en milieu éthanol en présence d'acide en un composé de formule générale VI:

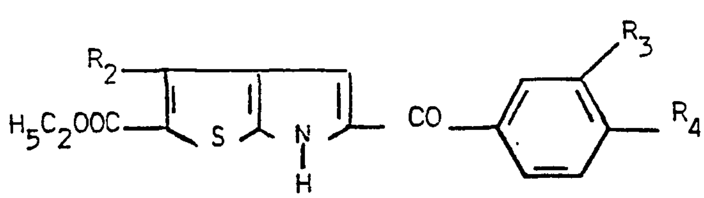

(VI)

dans laquelle $R_2$, $R_3$ et $R_4$ sont tels que définis précédemment,
que l'on traite tout d'abord par $NaOH/H_2O/C_2H_5OH$ puis par $HCl/H_2O$ pour donner le composé de formule générale VII:

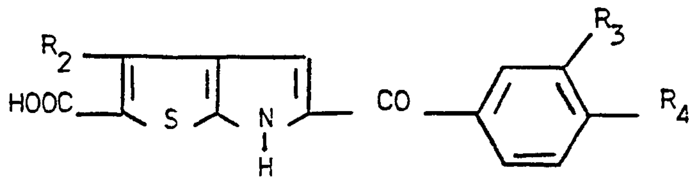

(VII)

dans laquelle $R_2$, $R_3$ et $R_4$ sont tels que définis précédemment, que l'on chauffe en présence de cuivre et de quinoléine pour obtenir le dérivé de formule générale VIII:

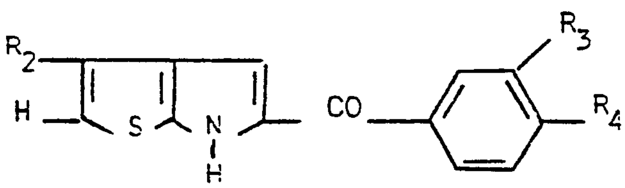

(VIII)

dans laquelle $R_2$, $R_3$ et $R_4$ ont les significations énoncées précédemment, que l'on traite en présence de $C_2H_5ONa/C_2H_5OH$, par un dérivé de formule générale IX:

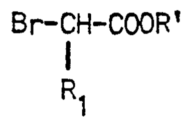

(IX)

dans laquelle $R_1$ a la signification énoncée précédemment et R' a la signification de R énoncée précédemment à l'exception d'un atome d'hydrogène, pour obtenir le dérivé de formule générale I':

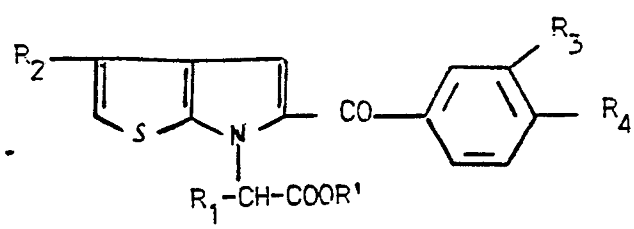

(I')

dans laquelle R', $R_1$, $R_2$, $R_3$ et $R_4$ sont tels que précédemment définis, lequel dérivé (I') est traité par $NaOH/H_2O/C_2H_5OH$ puis par une solution d'acide chlorhydrique pour obtenir le dérivé de formule générale I":

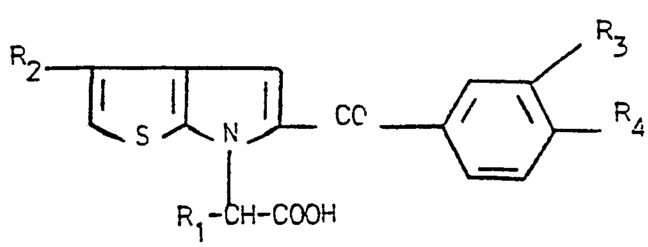

(I")

dans laquelle $R_1$, $R_2$, $R_3$ et $R_4$ sont tels que définis précédemment.

**Patentansprüche**

für die Vertragsstaaten BE, CH, DE, FR, GB, IT, LI, LU, NL, SE

1. Derivate des Thieno[2,3-b]pyrrols der allgemeinen Formel I

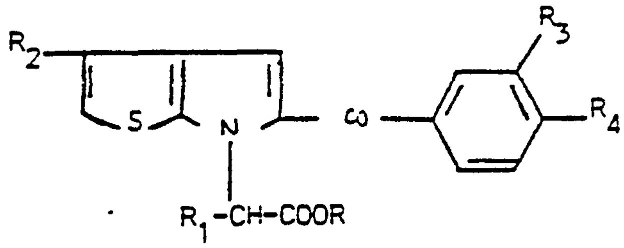

(I)

worin R ein Wasserstoffatom, eine geradkettige oder verzweigte Alkylgruppe mit 1 bis 5 Kohlenstoffatomen, ein Alkali- oder Erdalkalimetall, eine Ammoniumgruppe oder eine Mono-, Di-, oder Trialkylammoniumgruppe, in der die Alkylgruppe 1 bis 5 Kohlenstoffatome enthält,

$R_1$ ein Wasserstoffatom oder eine geradkettige oder verzweigte Alkylgruppe mit 1 bis 5 Kohlenstoffatomen,

$R_2$ ein Wasserstoffatom, eine geradkettige oder verzweigte Alkylgruppe mit 1 bis 5 Kohlenstoffatomen, eine Phenyl- oder Phenylalkylgruppe,in der die geradkettige oder verzweigte Alkylgruppe 1 bis 5 Kohlenstoffatome enthält,

$R_3$ und $R_4$, die gleich oder voneinander verschieden sind, jeweils Wasserstoff- oder Halogenatome oder geradkettige oderverzweigte alkyl- oder Alkoxygruppen mit 1 bis 5 Kohlenstoffatomen oder

$R_3$ und $R_3$ gemeinsam eine -CH=CH-CH=CH-Gruppe, so daß mit der Phenylgruppe, an die sie gebunden sind,eine Naphtylgruppe geblidet wird, bedeuten.

2. 3-Methyl-6-benzoyl-6-carboxymethyl-thieno[2,3-b]pyrrol.

3. 3-Methyl-6-benzoyl-6α-carboxyethyl-thieno[2,3-b]pyrrol.

4. 3-Methyl-5-p-methoxybenzoyl-6-carboxymethyl-thieno[2,3-b] pyrrol.

5. 3-Methyl-5-p-chlorbenzoyl-6a-carboxyethyl-thieno[2,3-b]pyrrol.

6. Verfahren zur Herstellung der Verbindungen nach Anspruch 1, dadurch gekennzeichnet, daß man ein Derivat der allgemeinen Formel II

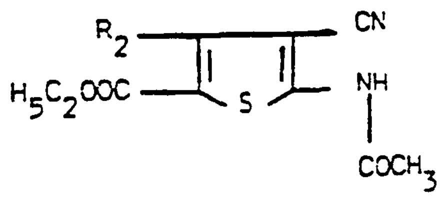

(II)

worin $R_2$ die Bedeutung wie in Anspruch 1, hat mit einem Derivat der Formel (III)

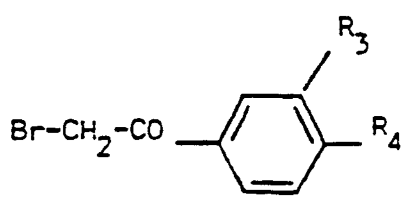

(III)

worin $R_3$ und $R_4$ die Bedeutungen wie in Anspruch 1 haben, in Gegenwart von $K_2CO_3$ und Aceton umsetzt, unter Bildung eines Derivats der allgemeinen Formel IV

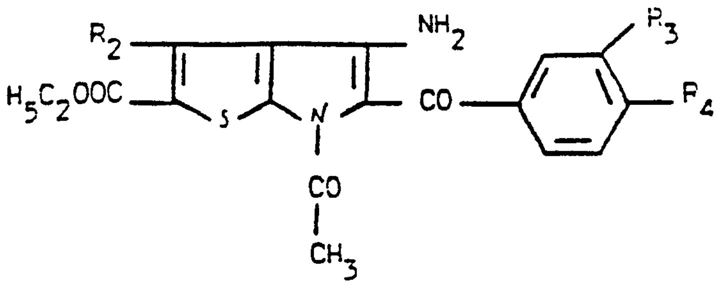

(IV)

worin $R_2$, $R_3$ und $R_4$ die gleichen Bedeutungen wie in Anspruch 1 haben, die Verbindung IV dann mit $KNO_2$, $HCl/H_2O$ und $CH_3OH$ unter Bildung der Verbindung der allgemeinen Formel V

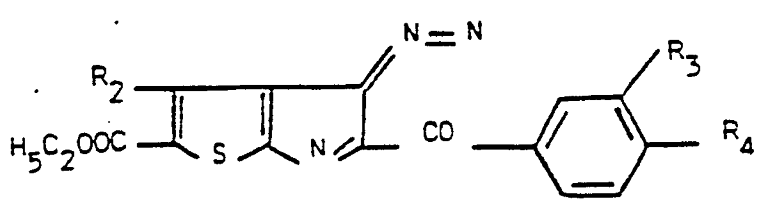

(V)

worin $R_2$, $R_3$ und $R_4$ die gleichen Bedeutungen wie in Anspruch 1 haben, behandelt, diese Verbindungen mittels Azabisisobutyronitril iim Ethanolmediumin G egenwart einer Säure zu der Verbindung der allgemeinen Formel VI

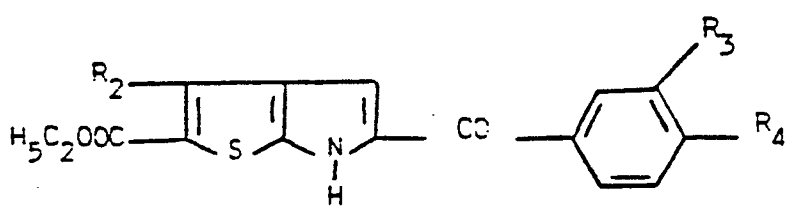

(VI)

worin $R_2$, $R_3$ und $R_4$ die gleichen Bedeutungen wie in Anspruch 1 haben, entdiazotiert,
zunächst mit $NaOH/H_2O/C_2H_5OH$ und dann m it $HCl/H_2O$ unter Bildung der Verbindung der allgemeinen Formel VII

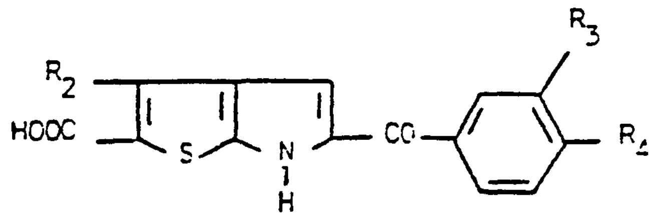

(VII)

worin $R_2$, $R_3$ und $R_4$ die gleichen Bedeutungen wie in Anspruch 1 haben, behandelt, in Gegenwart von Kupfer und Chinolin unter Bildung des Derivats der allgemeinen Formel VIII

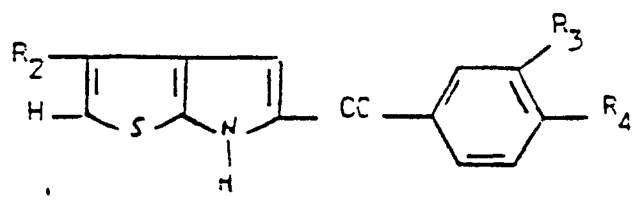

(VIII)

worin $R_2$, $R_3$ und $R_4$ die gleichen Bedeutungen wie in Anspruch 1 haben, erhitzt, in Gegenwart von $C_2H_5ONa/C_2H_5OH$ mit einem Derivat der all gemeinen Formel IX

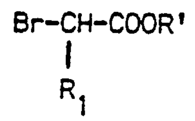

(IX)

worin $R_1$ die gleiche Bedeutung wie in Anspruch 1 und R' die gleiche Bedeutung wie R mit Ausnahme des Wasserstoffatoms haben behandelt, unter Bildung eines Derivats der allgemeinen Formel I'

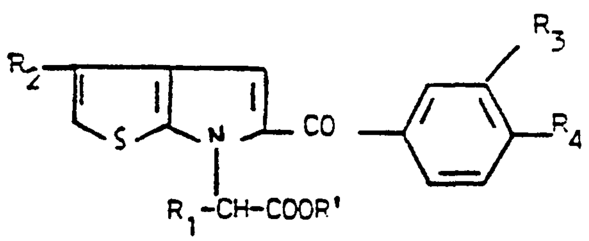

(I')

worin R', $R_1$, $R_2$, $R_3$ und $R_4$ die gleichen oben angegebenen Bedeutungen haben, umsetzt,
das Derivat (I') zunächst mit $NaOH/H_2O/C_2H_5OH$ und dann mit einer Chlorwasserstoffsäurelösung unter Bildung des Derivats derallgemeinen Formel I''

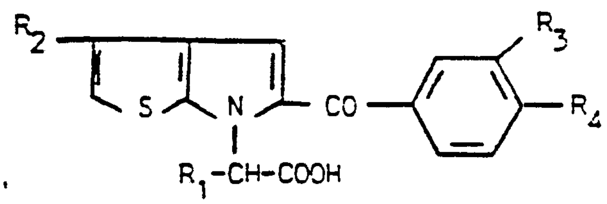

(I")

worin $R_1$, $R_2$, $R_3$ und $R_4$ die gleichen Bedeutungen wie in Anspruch 1 haben, umsetzt.

7. Pharmazeutische Zusarmmensetzungen, enthaltend als pharmazeutisch aktive Substanz eine Verbindung nach den Ansprüchen 1 bis 5 sowie geeignete Hilfsstoffe.

8. Pharmazeutische Zusammensetzungen nach Anspruch 7 zur Verwendung bei der Behandlung von akuten oder chronischen Schmerzen.

**Patentanspruch**

für den Vertragsstaat AT

Verfahren zur Herstellung von Derivaten des Thieno [2,3-b] pyrrols der allgemeinen Formel I

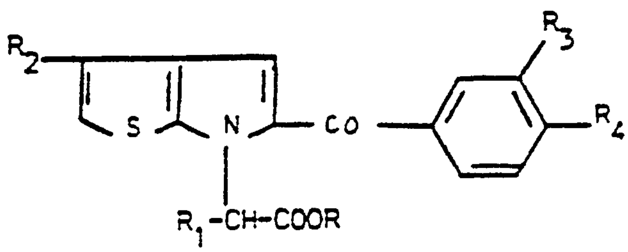

(I)

worin R ein Wasserstoffatom, eine geradkettige oder verzeeigte Alkylgruppe mit 1 bis 5 Kohlenstoffatomen, ein Alkali- oder Erdalkalimetall, eine Ammoniumgruppe, eine Mono-, Di-, oder Trialkylammoniumgruppe, in der die Alkylgruppe 1 bis 5 Kohlenstoffatome enthält,

$R_1$ ein Wasserstoffatom oder eine geradkettige oder verzweigte Alkylgruppe mit 1 bis 5 Kohlenstoffatornen,

$R_2$ ein Wasserstoffatom, eine geradkettige oder verzweigte Alkylgruppe mit 1 bis 5 Kohlenstoffatomen, eine Phenyl- oder Phenylalkylgruppe, in der die geradkettige oder verzweigte Alkylgruppe 1 bis 5 Kohlenstoffatome enthält,

$R_3$ und $R_4$, die gleich oder voneinander verschieden sind, Wasserstoff- oder Halogenatome oder geradkettige oder verzweigte Alkyl- oder Alkoxygruppen mit 1 bis 5 Kohlenstoffatomen oder

$R_3$ und $R_4$ gemeinsam eine - CH = CH - CH = CH -Gruppe,so daß mit der Phenylgruppe, an die sie gebunden sind, eine Naphtylgruppe gebildet wird, bedeuten, dadurch gekennzeichnet, daß man ein Derivat der allgemeinen Formel II

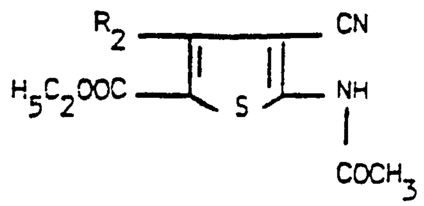

(II)

worin R2 die vorher angegebenen Bedeutungen hat, mit einem Derivat der Formel

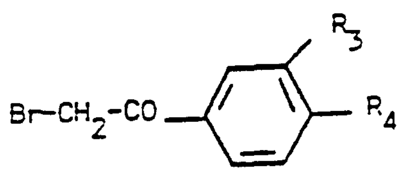

(III)

worin $R_3$ und $R_4$ die vorher angegebenen Bedeutungen haben, in Gegenwart von $K_2CO_3$ und Aceton umsetzt, unter Bildung eines Derivats der allgemeinen Formel IV

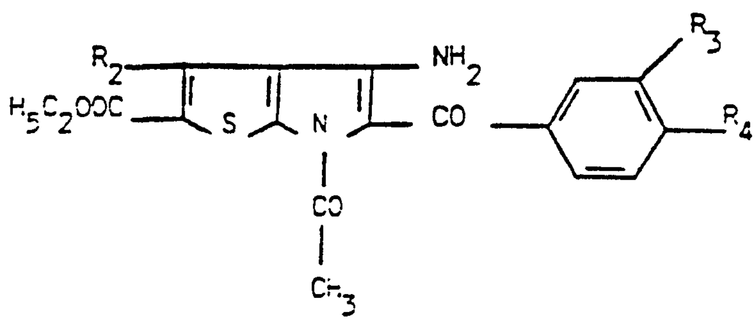

(IV)

worin $R_2$,$R_3$ und $R_4$ die vorherangegebenen Bedeutungen haben, die Verbindung IV dann mit $KNO_2$, $HCl/H_2O$ und $CH_3OH$ unter Bildung der Verbindung der allgemeinen Formel V

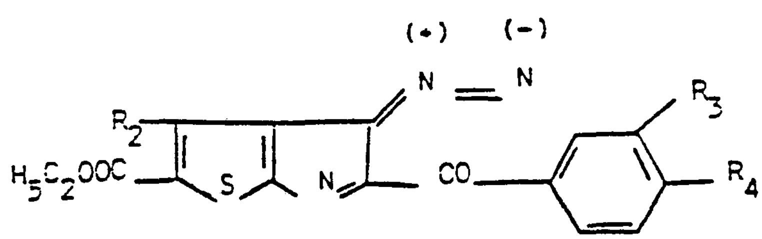

(V)

worin $R_2$,$R_3$ und $R_4$ die vorherangegebenen Bedeutungen haben,behandelt, diese Verbindung mittels Azabisisobutyronitrilim Ethanolmedium in G egenwart einer Säure zu einer Verbindung der allgemeinen Formel VI

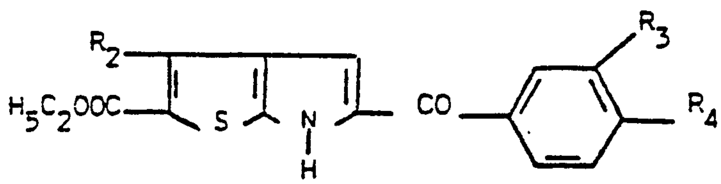

(VI)

worin $R_2$, $R_3$ und $R_4$ die vorherangegebenen Bedeutungen haben, entdiazotiert, zunächst mit $NaOH/H_2O/C_2H_5OH$ und dann mit $HCl/H_2O$ unter Bildung der Verbindung der allgemeinen Formel VII

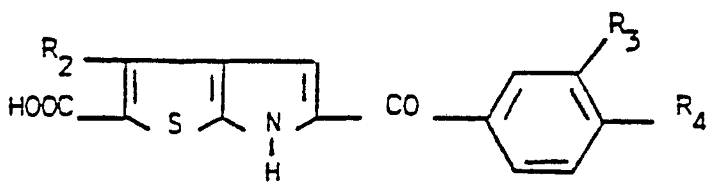

(VII)

worin $R_2$, $R_3$ und $R_4$ die vorherangegebenen Bedeutungen haben, behandelt, in Gegenwart von Kupfer und Chinolin unter Bildung des Derivats der allgemeinen Formel VIII

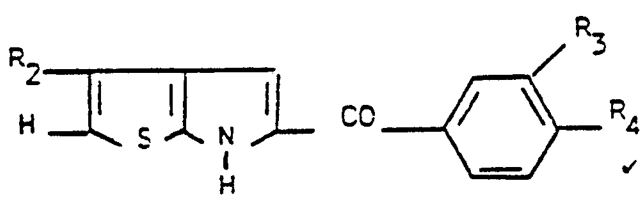

(VIII)

worin $R_2$, $R_3$ und $R_4$ die vorherangegebenen Bedeutungen haben, erhitzt, in Gegenwart von $C_2H_5ONa/C_2H_5OH$ mit einem Derivat der allgemeinen Formel IX

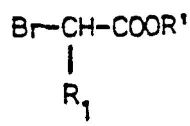

(IX)

worin R1 die vorher angegebene Bedeutung und R'die gleiche Bedeutung wie R mit Ausnahme des Wasserstoffatoms haben unter Bildung eines Derivats derallgemeinen Formel I'

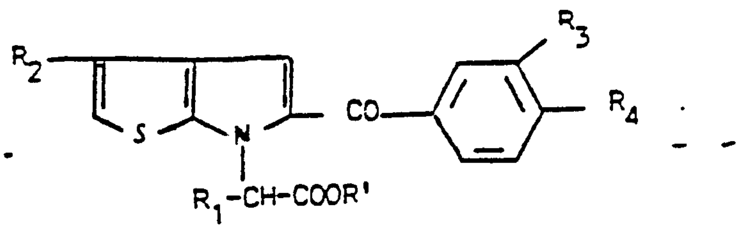

(I')

worin R', R1, R2, R3 und R4 die gleichen oben angegebenen Bedeutungen haben, umsetzt, das Derivat (I') zunächst mit $NaOH/H_2O/C_2H_5OH$ und dann mit einer Chlorwasserstoffsäurelösung unter Bildung des Derivats der allgemeinen Formel I'

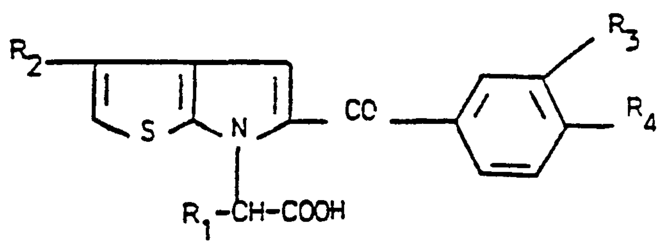

(I")

worin $R_1$, $R_2$, $R_3$ und $R_4$ die vorherangegebenen Bedeutungen haben, behandelt.

**Claims**

for the contracting states BE, CH, DE, FR, GB, IT, LI, LU, NL, SE

1. Derivatives of thieno[2,3-b]pyrrole of the general formula I:

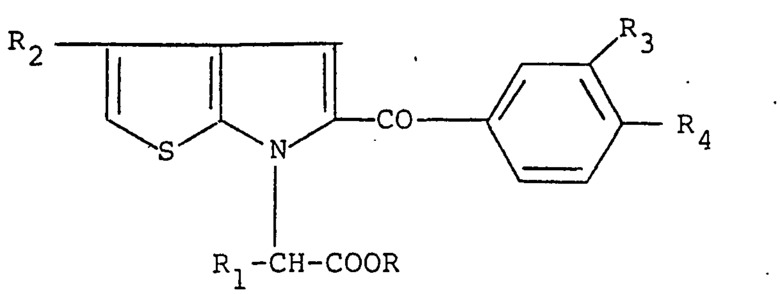

(I)

in which

R represents a hydrogen atom, a straight- or branched-chain alkyl radical having from 1 to 5 carbon atoms, an alkali metal or an alkaline earth metal, or an ammonium or mono-, di- or tri-alkylammonium radical in which the alkyl group contains from 1 to 5 carbon atoms,

$R_1$ represents a hydrogen atom or a straight- or branched-chain alkyl radical containing from 1 to 5 carbon atoms,

$R_2$ represents a hydrogen atom, a straight- or branched-chain alkyl radical having from 1 to 5 carbon atoms, a phenyl radical or a phenylalkyl radical in which the alkyl group contains from 1 to 5 carbon atoms in a straight or branched chain, $R_3$ and $R_4$, which may be the same or different, each represents a hydrogen or halogen atom or a straight- or branched-chain alkyl or alkoxy radical each having from 1 to 5 carbon atoms, or $R_3$ and $R_4$

together represent a radical of the formula -CH=CH-CH=CH- so as to form, with the phenyl group to which they are bonded, a naphthyl group.

2. 3-methyl-5-benzoyl-6-carboxymethylthieno[2,3-b]-pyrrole.

3. 3-methyl-5-benzoyl-6-(α-carboxyethyl)-thieno-[2,3-b]pyrrole.

4. 3-methyl-5-(p-methoxybenzoyl)-6-carboxymethyl-thieno[2,3-b]pyrrole.

5. 3-methyl-5-(p-chlorobenzoyl)-6-(α-carboxyethyl)-thieno[2,3-b]pyrrole.

6. Process for the preparation of the compounds of claim 1, characterised in that:
the derivative of the general formula II:

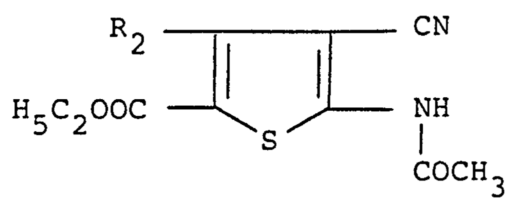

(II)

in which $R_2$ has the meaning given in claim 1, is treated with a derivative of the general formula III:

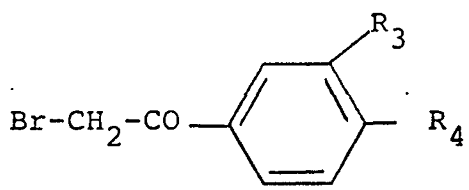

(III)

in which $R_3$ and $R_4$ have the meanings given in claim 1, in the presence of $K_2CO_3$ and acetone, to obtain the derivative of the general formula IV:

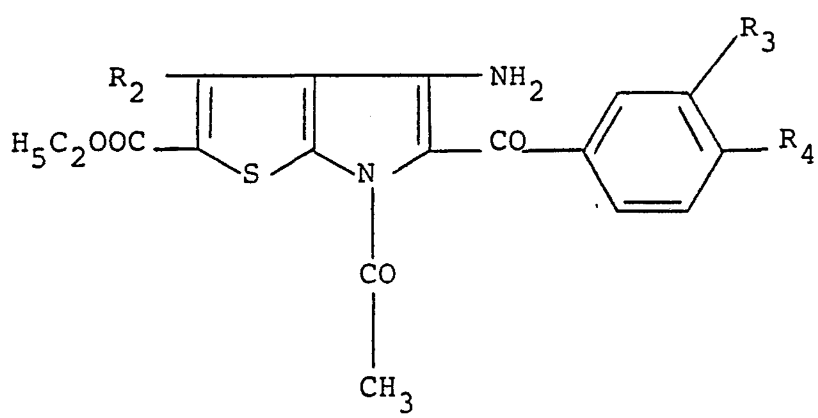

(IV)

in which $R_2$, $R_3$ and $R_4$ are as defined in claim 1, which compound IV is treated with $KNO_2$, $HCl/H_2O$ and $CH_3OH$ to obtain the compound of the general formula V:

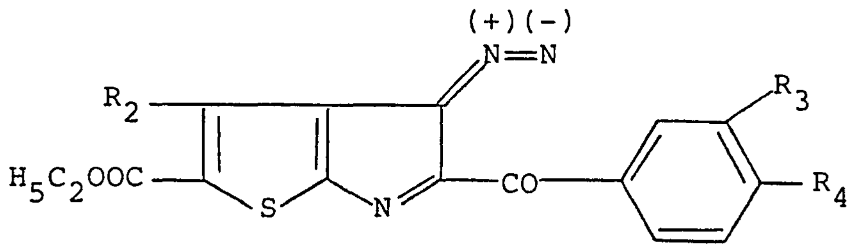

(V)

in which $R_2$, $R_3$ and $R_4$ have the meanings defined in claim 1,
which compound is de-diazotised by means of azabisisobutyronitrile in an ethanol medium, in the presence of an acid, to form a compound of the general formula VI:

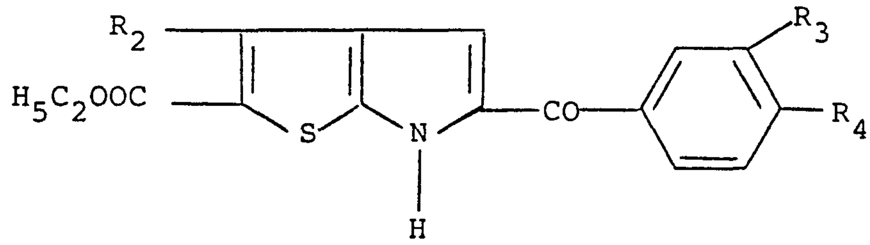

(VI)

in which $R_2$, $R_3$ and $R_4$ are as defined in claim 1 which compound is treated firstly with $NaOH/H_2O/C_2H_5OH$ and then with $HCl/H_2O$ to give the compound of the general formula VII:

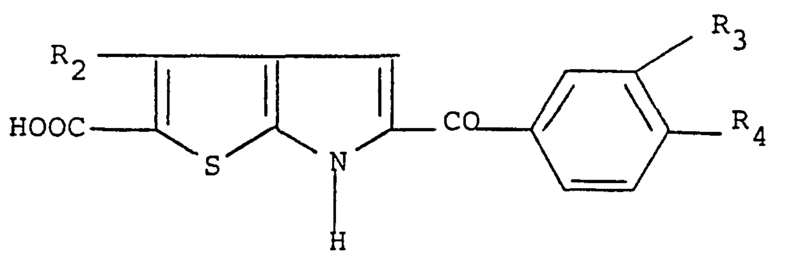

(VII)

in which $R_2$, $R_3$ and $R_4$ are as defined in claim 1, which compound is heated in the presence of copper and quinoline to obtain the derivative of the general formula VIII:

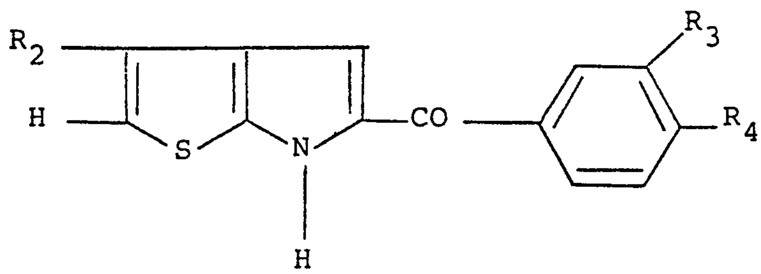

(VIII)

in which $R_2$, $R_3$ and $R_4$ have the meanings given in claim 1,
which derivative is treated, in the presence of $C_2H_5ONa/C_2H_5OH$, with a derivative of the general formula IX:

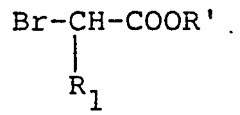

(IX)

in which $R_1$ has the meaning given in claim 1 and R' has the meaning given for R in claim 1, with the exception of a hydrogen atom,
to obtain the derivative of the general formula I':

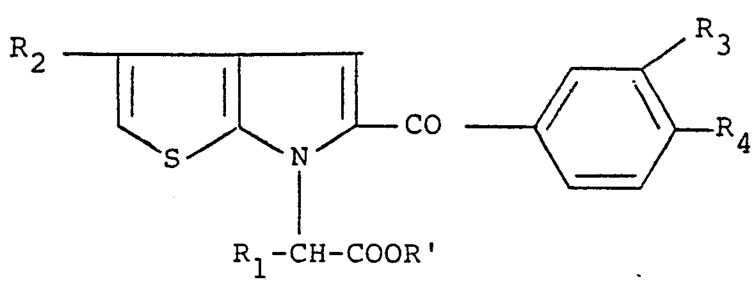

(I')

in which R', $R_1$, $R_2$, $R_3$ and $R_4$ are as defined above which derivative (I') is treated with $NaOH/H_2O/C_2H_5OH$ and then with a solution of hydrochloric acid to obtain the derivative of the general formula I":

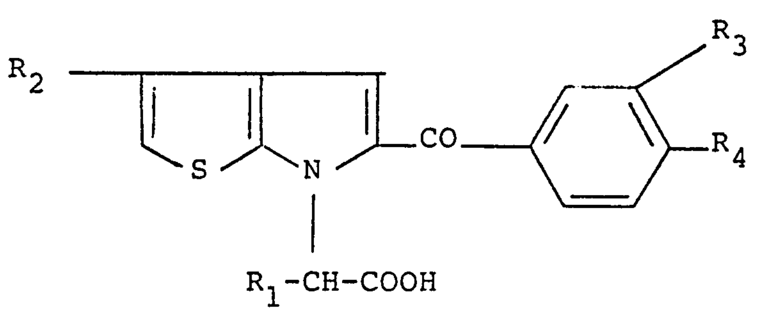

(I")

in which $R_1$, $R_2$, $R_3$ and $R_4$ are as defined in claim 1.

7. Pharmaceutical compositions containing as active ingredient a compound according to claims 1 to 5 with appropriate excipients.

8. Pharmaceutical compositions according to claim 7 for use in the treatment of acute or chronic pain.

**Claim**

for the contracting state AT

Process for the preparation of derivatives of thieno[2,3-b]pyrrole of the general formula I:

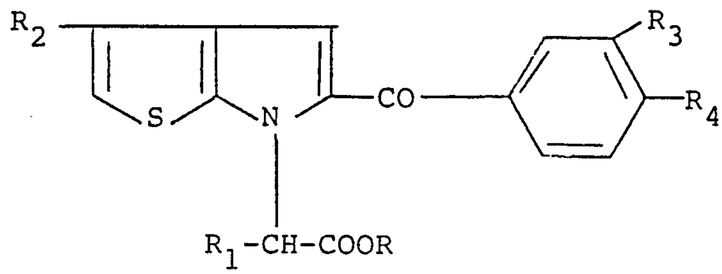

(I)

in which

R represents a hydrogen atom, a straight- or branched-chain alkyl radical having from 1 to 5 carbon atoms, an alkali metal or an alkaline earth metal, or an ammonium or mono-, di- or tri-alkylammonium radical in which the alkyl group contains from 1 to 5 carbon atoms,

$R_1$ represents a hydrogen atom or a straight- or branched-chain alkyl radical containing from 1 to 5 carbon atoms,

$R_2$ represents a hydrogen atom, a straight- or branched-chain alkyl radical having from 1 to 5 carbon atoms, a phenyl radical or a phenylalkyl radical in which the alkyl group contains from 1 to 5 carbon atoms in a straight or branched chain, $R_3$ and $R_4$, which may be the same or different, each represents a hydrogen or halogen atom or a straight- or branched-chain alkyl or alkoxy radical each having from 1 to 5 carbon atoms, or

$R_3$ and $R_4$ together represent a radical of the formula -CH=CH-CH=CH- so as to form, with the phenyl group to which they are bonded, a naphthyl group,

characterised in that:

the derivative of the general formula II:

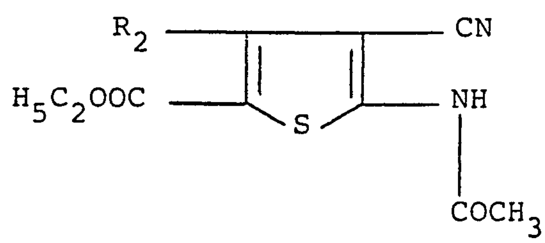

(II)

in which $R_2$ has the meaning given above, is treated with a derivative of the general formula III:

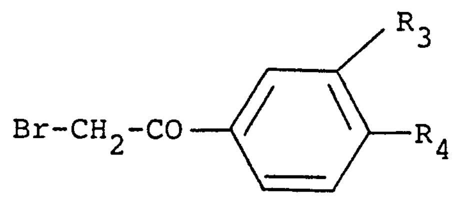

(III)

in which $R_3$ and $R_4$ have the meanings given above, in the presence of $K_2CO_3$ and acetone, to obtain the derivative of the general formula IV:

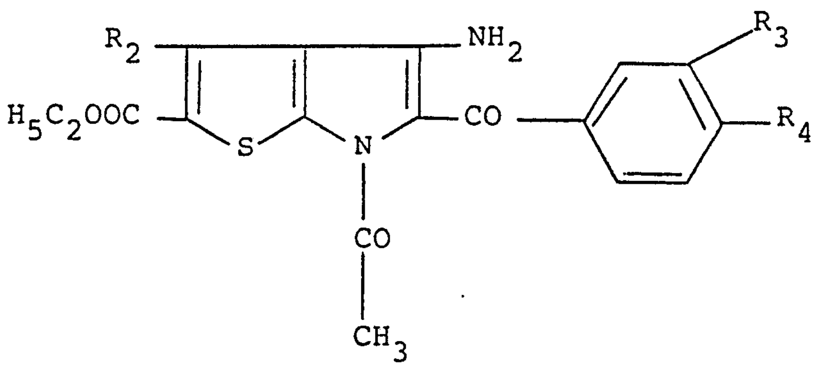

(IV)

in which $R_2$ $R_3$ and $R_4$ are as defined above, which compound IV is treated with $KNO_2$, $HCl/H_2O$ and $CH_3OH$ to obtain the compound of the general formula V:

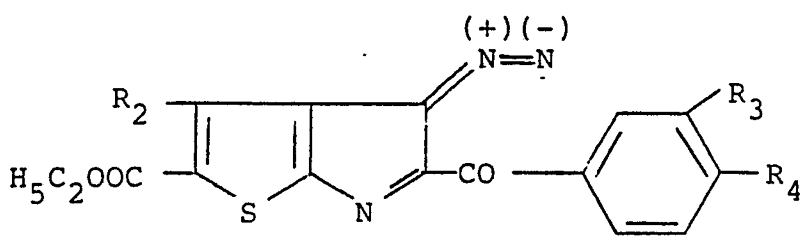

(V)

in which $R_2$, $R_3$ and $R_4$ have the meanings defined above,

which compound is de-diazotised by means of azabisisobutyronitrile in an ethanol medium, in the presence of an acid, to form a compound of the general formula VI:

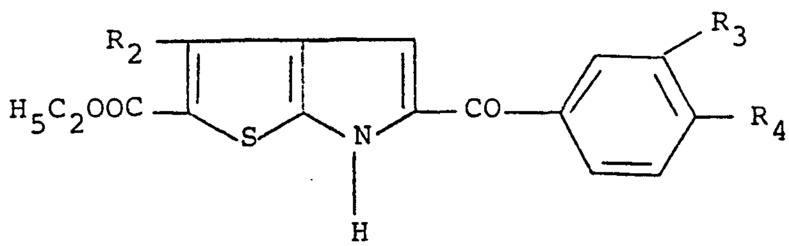

(VI)

in which $R_2$, $R_3$ and $R_4$ are as defined above, which compound is treated firstly with $NaOH/H_2O/C_2H_5OH$ and then with $HCl/H_2O$ to give the compound of the general formula VII:

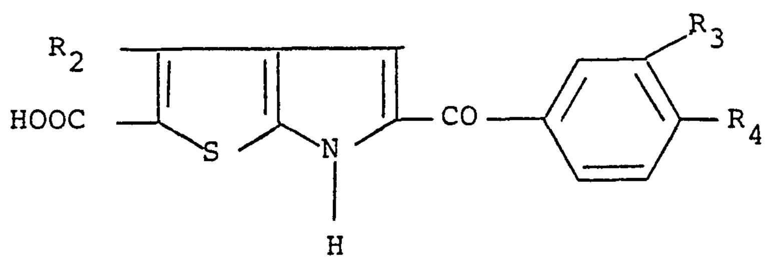

(VII)

in which $R_2$, $R_3$ and $R_4$ are as defined above, which compound is heated in the presence of copper and quinoline to obtain the derivative of the general formula VIII:

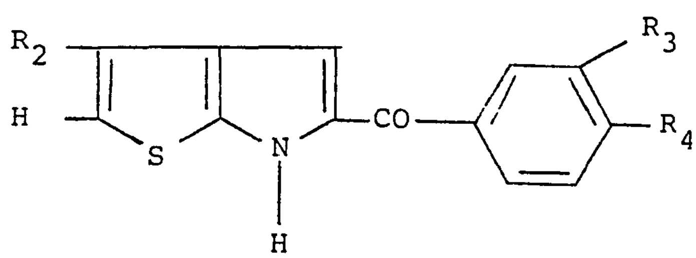

(VIII)

in which $R_2$, $R_3$ and $R_4$ have the meanings given above,
which derivative is treated, in the presence of $C_2H_5ONa/C_2H_5OH$, with a derivative of the general formula IX:

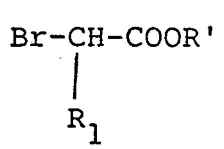

(IX)

in which $R_1$ has the meaning given above and R′ has the meaning given for R above, with the exception of a hydrogen atom,
to obtain the derivative of the general formula I′:

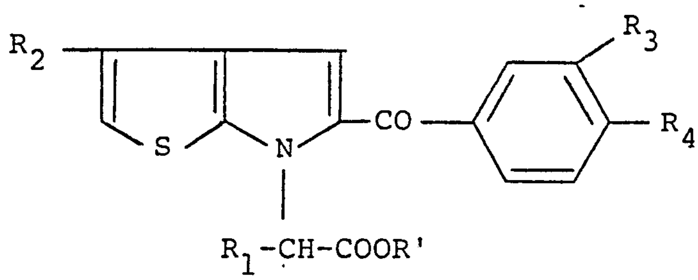

(I')

in which R', $R_1$, $R_2$, $R_3$ and $R_4$ are as defined above, which derivative (I') is treated with $NaOH/H_2O/C_2H_5OH$ and then with a solution of hydrochloric acid to obtain the derivative of the general formula I'':

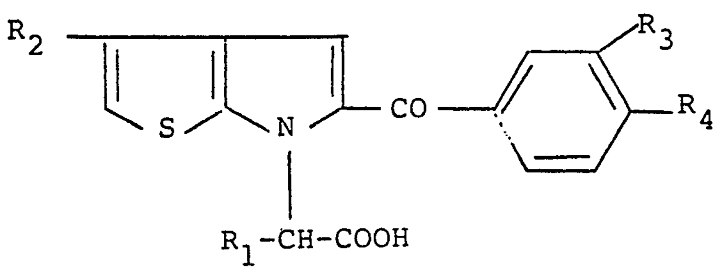

(I")

in which $R_1$, $R_2$ $R_3$ and $R_4$ are as defined above.